# EUROPEAN PATENT APPLICATION

(11) **EP 2 883 501 A2**
(43) Date of publication of application: **17.06.2015**
(21) Application number: 14173038.2
(22) Date of filing: 18.06.2014
(51) Int. Cl.: A61B 8/08, A61B 8/00

(54) **Ultrasound diagnosis device and operating method of the same**

(30) Priority: 16.12.2013 KR 20130156644
(71) Applicant: Samsung Medison Co., Ltd., Gangwon-do 250-870 (KR)
(72) Inventor: Lee, Jin-yong, Gangwon-do (KR); Park, Sung-wook, Gangwon-do (KR); Song, Joo-hyun, Gangwon-do (KR); Lee, Bong-heon, Gangwon-do (KR); Chang, Hyuk-jae, Seoul (KR); Chung, Nam-sik, Seoul (KR); Hong, Geu-ru, Seoul (KR); Kim, Jong-hwa, Seoul (KR); Shin, Sang-hoon, Seoul (KR)
(74) Representative: Schmid, Wolfgang

(57) **Abstract**

Disclosed are an ultrasound diagnosis device and an operating method of the same. The operating method of an ultrasound diagnosis device includes generating a first ultrasound image based on first ultrasound signals received from an object at a first point in time, calculating a value of at least one parameter of the object based on the first ultrasound image, generating a second ultrasound image based on second ultrasound signals received from the object at a second point in time, calculating a value of the at least one parameter of the object based on the second ultrasound image, and displaying a comparative image obtained by comparing the first and second ultrasound images and a comparative result obtained by comparing the calculated values of the at least one parameter.

## Description

### RELATED APPLICATIONS

This application claims the benefit of Korean Patent Application No. 10-2013-0156644, filed on December 16, 2013, in the Korean Intellectual Property Office, the disclosure of which is incorporated herein in its entirety by reference.

### BACKGROUND

### 1. Field

One or more embodiments of the present invention relate to an ultrasound diagnosis device and an operating method of the same, and more particularly, to an ultrasound diagnosis device for accurately and readily diagnosing a state change of an object and an operating method of the ultrasound diagnosis device.

### 2. Description of the Related Art

An ultrasound diagnosis device emits ultrasound signals generated by transducers of a probe to an object and receives echo signals reflected from the object, thereby obtaining images regarding the interior of the object. Particularly, an ultrasound diagnosis device may be used for medical purposes including observation of the interior of an object, detection of foreign substances, and diagnosis of damage. Such an ultrasound diagnosis device is stabler than a diagnosis device using X-ray and safe without causing radioactive exposure, and may display an image in real time. Therefore, ultrasound diagnosis devices are widely used together with other types of image diagnosis devices.

Meanwhile, an ultrasound diagnosis device may provide a brightness (B) mode in which reflection coefficients of ultrasound signals reflected from an object is shown as a two-dimensional (2D) image, a Doppler mode in which an image of a moving object (in particular, blood flow) is shown by using the Doppler effect, an elastic mode in which a difference in reaction between a case where an object is compressed and a case where the object is not compressed is shown as an image, and so on.

### SUMMARY

One or more embodiments of the present invention include an ultrasound diagnosis device which displays a comparative image obtained by comparing ultrasound images of an object and a comparative result obtained by comparing parameter values and thus may accurately and readily diagnose a state change of the object, and an operating method of the ultrasound diagnosis device.

Additional aspects will be set forth in part in the description which follows and, in part, will be apparent from the description, or may be learned by practice of the presented embodiments.

According to one or more embodiments of the present invention, an operating method of an ultrasound diagnosis device includes generating a first ultrasound image based on first ultrasound signals received from an object at a first point in time, calculating a value of at least one parameter of the object based on the first ultrasound image, generating a second ultrasound image based on second ultrasound signals received from the object at a second point in time, calculating a value of the at least one parameter of the object based on the second ultrasound image, and displaying a comparative image obtained by comparing the first and second ultrasound images and a comparative result obtained by comparing the calculated values of the at least one parameter.

The generating of the second ultrasound image may include generating the second ultrasound image based on the second ultrasound signals received from the object to which a therapy has been applied.

When the object is a heart, the therapy may be cardiac resynchronization therapy (CRT).

The at least one parameter may include at least one of a strain, a volume, a strain rate (SR), and a displacement.

The first and second points in time may be a same point in time in periods of the object.

The operating method may further include storing the first ultrasound image and the value of the at least one parameter calculated based on the first ultrasound image. Here, the generating of the second ultrasound image may include receiving the second ultrasound signals and generating the second ultrasound image in real time, and the displaying of the comparative image and the comparative result may include displaying a comparative image obtained by comparing the stored first ultrasound image and the second ultrasound image generated in real time and a comparative result obtained by comparing the stored value of the at least one parameter and the value of the at least one parameter calculated based on the second ultrasound image.

The generating of the first ultrasound image may include generating a three-dimensional (3D) ultrasound image by performing surface rendering, and the generating of the second ultrasound image may include generating a 3D ultrasound image by performing volume rendering.

The displaying of the comparative image and the comparative result may include displaying the first and second ultrasound images to overlap each other, and displaying a difference area between the first and second ultrasound images to be differentiated.

The displaying of the comparative image and the comparative result may include displaying the difference area in a different color from other areas.

The displaying of the comparative image and the comparative result may include displaying the calculated values of the at least one parameter in at least one of graphs, values, and colors.

The displaying of the comparative image and the comparative result may include displaying whether or not the value of the at least one parameter calculated based on the second ultrasound image is within a normal range.

According to one or more embodiments of the present invention, an ultrasound diagnosis device includes an image generating unit which generate a first ultrasound image based on first ultrasound signals received from an object at a first point in time and a second ultrasound image based on second ultrasound signals received from the object at a second point in time, a parameter calculating unit which calculates a value of at least one parameter based on the first ultrasound image and calculates a value of the at least one parameter based on the second ultrasound image, and a display unit which displays a comparative image obtained by comparing the first and second ultrasound images, and displays a comparative result obtained by comparing the values of the at least one parameter.

The image generating unit may generate the second ultrasound image based on the second ultrasound signals received from the object to which a therapy has been applied.

When the object is a heart, the therapy may be CRT.

The ultrasound diagnosis device may further include a memory which stores the first ultrasound image and the value of the at least one parameter calculated based on the first ultrasound image. Here, the image generating unit may receive the second ultrasound signals and generate the second ultrasound image in real time, and the display unit may display a comparative image obtained by comparing the stored first ultrasound image and the second ultrasound image generated in real time and a comparative result obtained by comparing the stored value of the at least one parameter and the value of the at least one parameter calculated based on the second ultrasound image.

The image generating unit may generate a 3D first ultrasound image by performing surface rendering, and generate a 3D second ultrasound image by performing volume rendering.

The display unit may display the first and second ultrasound images to overlap each other, and display a difference area between the first and second ultrasound images to be differentiated.

The display unit may display the difference area in a different color from other areas.

The display unit may display the calculated values of the at least one parameter in at least one of graphs, values, and colors.

The display unit may display whether or not the value of the at least one parameter calculated based on the second ultrasound image is within a normal range.

### BRIEF DESCRIPTION OF THE DRAWINGS

These and/or other aspects will become apparent and more readily appreciated from the following description of the embodiments, taken in conjunction with the accompanying drawings in which:
FIG. 1 is a block diagram showing a configuration of an ultrasound diagnosis device according to an embodiment of the present invention;
FIG. 2 is a block diagram showing a configuration of an ultrasound diagnosis device according to an embodiment of the present invention;
FIG. 3 is a flowchart of an operating method of an ultrasound diagnosis device according to an embodiment of the present invention; and
FIGS. 4 to 6 are diagrams for illustrating the operating method of FIG. 3.

### DETAILED DESCRIPTION

The terms used in this specification are those general terms currently widely used in the art in consideration of functions in regard to the present invention, but the terms may vary according to the intention of those of ordinary skill in the art, precedents, or new technology in the art. Also, specified terms may be selected by the applicant, and in this case, the detailed meaning thereof will be descried in the detailed description of the invention. Thus, the terms used in the specification should be understood not as simple names but based on the meaning of the terms and the overall description of the invention.

Throughout the specification, it will also be understood that when a component "includes" an element, unless there is another opposite description thereto, it should be understood that the component does not exclude another element but may further include another element. In addition, terms such as "... unit", "... module", or the like refer to units that perform at least one function or operation, and the units may be implemented as hardware or software or as a combination of hardware and software.

Throughout the specification, an "ultrasound image" refers to an image of an object obtained by using an ultrasound wave. Furthermore, the "object" may include a person or an animal, or a part of a person or an animal. For example, the object may include an organ, such as the liver, the heart, the womb, the brain, a breast, the abdomen, etc., or a blood vessel. Furthermore, the "object" may include a phantom. The phantom means a material having a volume that is approximately the density and effective atomic number of a living thing, and may include a sphere phantom having a property similar to a human body.

An ultrasound image may be variously implemented. For example, an ultrasound image may be at least one of an amplitude (A) mode image, a brightness (B) mode image, a color (C) mode image, and a Doppler (D) mode image. According to an embodiment of the present invention, an ultrasound image may be two-dimensional (2D) image or a three-dimensional (3D) image.

Throughout the specification, a "user" refers to a medical professional, such as a doctor, a nurse, a clinical pathologist, or a medical image expert, or an engineer who repairs a medical apparatus, but the user is not limited thereto.

Hereinafter, embodiments of the present invention will be described more fully with reference to the accompanying drawings so that those of ordinary skill in the art to which the present invention pertains may readily implement the embodiments. However, the present invention may be embodied in many different forms and should not be construed as being limited to the embodiments set forth herein. Expressions such as "at least one of," when preceding a list of elements, modify the entire list of elements and do not modify the individual elements of the list.

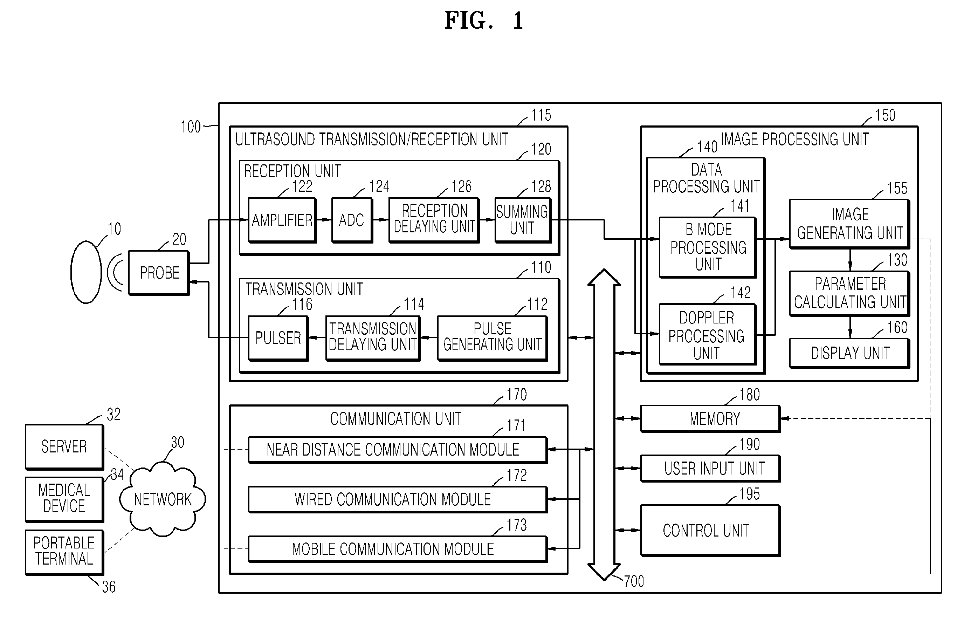

FIG. 1 is a block diagram showing a configuration of an ultrasound diagnosis device 100 according to an embodiment of the present invention.

The ultrasound diagnosis device 100 according to an embodiment of the present invention may include a probe 20, an ultrasound transmission/reception unit 115, an image processing unit 150, a communication unit 170, a memory 180, a user input unit 190, and a control unit 195, where the components mentioned above may be connected to one another via a bus 185. In addition, the image processing unit 150 may include an image generating unit 155, a parameter calculating unit 130, and a display unit 160.

The ultrasound diagnosis device 100 according to an embodiment of the present invention may be embodied not only as a cart type device, but also as a portable device. Examples of portable ultrasound diagnosis devices may include a picture archiving and communications system (PACS) viewer, a smart phone, a laptop computer, a personal digital assistant (PDA), and a tablet PC, but portable ultrasound diagnosis devices are not limited thereto.

The probe 20 transmits ultrasound signals to an object 10 according to a driving signal applied from the ultrasound transmission/reception unit 115 and receives echo signals reflected by the object 10. The probe 20 includes a plurality of transducers, and the plurality of transducers oscillate according to electric signals transmitted thereto and generate acoustic energy, that is, ultrasound waves. The probe 20 may be connected to the main body of the ultrasound diagnosis device 100 wiredly or wirelessly, and the ultrasound diagnosis device 100 may include a plurality of probes 20.

A transmission unit 110 supplies the driving signal to the probe 20, and includes a pulse generating unit 112, a transmission delaying unit 114, and a pulser 116. The pulse generating unit 112 generates pulses for forming transmission ultrasound waves according to a predetermined pulse repetition frequency (PRF), and the transmission delaying unit 114 applies delay times for determining transmission directionality to the pulses. Pulses to which the delay times are applied correspond to a plurality of piezoelectric vibrators included in the probe 20, respectively. The pulser 116 applies the driving signal (or driving pulse) to the probe 20 at a timing corresponding to each pulse to which a delay time is applied.

A reception unit 120 generates ultrasound data by processing the echo signals received from the probe 20, and may include an amplifier 122, an analog-digital converter (ADC) 124, a reception delaying unit 126, and a summing unit 128. The amplifier 122 amplifies the echo signals in each channel, and the ADC 124 analog-digital converts the amplified echo signals. The reception delaying unit 126 applies delay times for determining reception directionality to the digital-converted echo signals, and the summing unit 128 generates ultrasound data by summing the echo signals processed by the reception delaying unit 126.

The image processing unit 150 generates an ultrasound image by scan-converting the ultrasound data generated by the ultrasound transmission/reception unit 115 and displays the ultrasound image.

Meanwhile, an ultrasound image may be a grayscale ultrasound image obtained by scanning an object in the A mode, the B mode, and a motion (M) mode, and may also show motion of an object as a Doppler image. A Doppler image may include a blood flow Doppler image showing flow of blood (aka a color Doppler image), a tissue Doppler image showing movement of tissues, and a spectral Doppler image showing moving speed of an object as a waveform.

A B mode processing unit 141 extracts B mode components from the ultrasound data and processes the B mode components. The image generating unit 155 may generate an ultrasound image indicating signal intensities as brightness based on the extracted B mode components.

Similarly, a Doppler processing unit 142 may extract Doppler components from the ultrasound data, and the image generating unit 155 may generate a Doppler image indicating movement of the object 10 as colors or waveforms based on the extracted Doppler components.

The image generating unit 155 according to an embodiment of the present invention may generate a 3D ultrasound image via surface rendering or volume rendering of volume data and may also generate an elasticity image which visualizes deformation of the object 10 due to a pressure. Furthermore, the image generating unit 155 may display various additional information in an ultrasound image by using texts and graphics. Meanwhile, the generated ultrasound image may be stored in the memory 180.

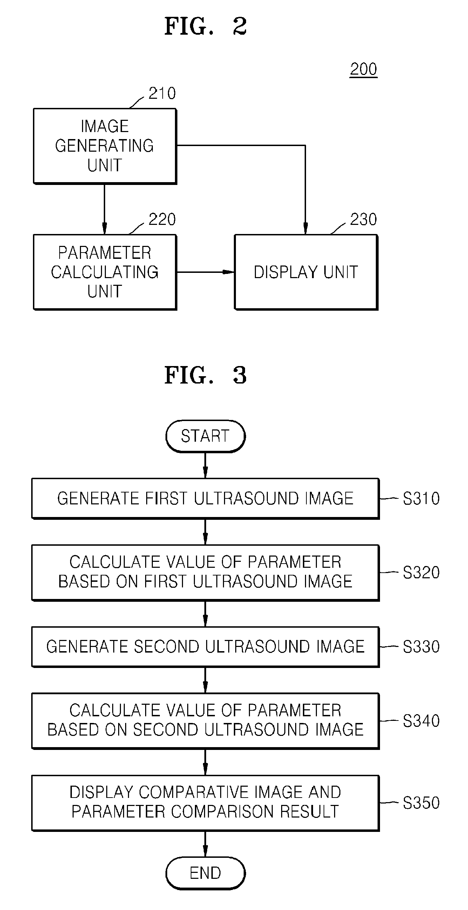

The parameter calculating unit 130 may calculate a value of at least one parameter of the object 10 based on the ultrasound image generated by the image generating unit 155 and store the calculated parameter value in the memory 180. This will be described in detail in FIG. 2.

The display unit 160 displays the generated ultrasound image. The display unit 160 may display not only an ultrasound image, but also various information processed by the ultrasound diagnosis device 100 on a screen via a graphic user interface (GUI). Meanwhile, the ultrasound diagnosis device 100 may include two or more display units 160 according to embodiments.

The display unit 160 may include at least one of a liquid crystal display (LCD), a thin film transistor-liquid crystal display (TFT-LCD), an organic light-emitting diode (OLED), a flexible display, a 3D display, and an electrophoretic display.

When the display unit 160 and the user input unit 190 constitute a layer structure and are configured as a touch screen, the display unit 160 may be used as an input device to which information may be input by a touch of a user in addition to an output device.

The touch screen may be configured to detect a position of a touch input, a touched area, and also a touch pressure. Also, the touch screen may be configured to detect a proximity touch as well as a real touch.

In this specification, a "real touch" refers to a case where a pointer actually touches a screen, and a "proximity touch" refers to a case where a pointer does not actually touches a screen but approaches the screen at a predetermined distance. In this specification, a pointer refers to a touch tool for touching or proximity-touching a particular portion of a displayed screen. Examples of a pointer may be an electronic pen, a finger, and so on.

Although not shown in the drawing, the ultrasound diagnosis device 100 may include various sensors in order to sense a real touch or a proximity touch on the touch screen. An example of a sensor for sensing a touch on the touch screen is a tactile sensor.

A tactile sensor refers to a sensor which senses a contact of a particular object to the degree of human sensitivity or higher. A tactile sensor may sense various information, such as the toughness of a contact surface, the hardness of a contact object, the temperature of a contact point, and so on.

Another example of a sensor for sensing a touch on the touch screen is a proximity sensor. A proximity sensor refers to a sensor which detects an object approaching a predetermined detection surface or an object present thereabout by using the strength of an electromagnetic field or infrared light without a mechanical contact.

Examples of proximity sensors include a transmissive photoelectric sensor, a direct reflective photoelectric sensor, a mirror reflective photoelectric sensor, a highfrequency oscillation proximity sensor, a capacitive proximity sensor, a magnetic proximity sensor, an infrared proximity sensor, and so on.

The communication unit 170 is wiredly or wirelessly connected to a network 30 and communicates with an external device or a server. The communication unit 170 may exchange data with a hospital server or another medical device in a hospital that is connected through a PACS. Also, the communication unit 170 may perform data communication according to the digital imaging and communications in medicine (DICOM) standard.

The communication unit 170 may transmit and receive data related to diagnosis of the object 10, such as an ultrasound image, ultrasound data, and Doppler data of the object 10, via the network 30 and may also transmit and receive medical images obtained via other medical devices, such as, a computer tomography (CT) image, a magnetic resonance (MR) image, and an X-ray image. In addition, the communication unit 170 may receive information related to a diagnosis history, a treatment schedule, etc. of a patient from a server and utilize the information for diagnosing the patient. Furthermore, the communication unit 170 may perform data communication not only with a server or a medical device in a hospital, but also with a portable terminal of a doctor or a patient.

The communication unit 170 is connected to the network 30 wiredly or wirelessly and may exchange data with a server 32, a medical device 34, or a portable terminal 36. The communication unit 170 may include one or more components that enable communication with external devices, e.g., a close-distance communication module 171, a wired communication module 172, and a mobile communication module 173.

The near distance communication module 171 refers to a module for near distance communication within a predetermined distance. Examples of near distance communication techniques according to an embodiment of the present invention may include wireless LAN, Wi-Fi, Bluetooth, Zigbee, Wi-Fi Direct (WFD), ultra wideband (UWB), infrared data association (IrDA), Bluetooth Low Energy (BLE), and near field communication (NFC), but near distance communication techniques according to an embodiment of the present invention are not limited thereto.

The wired communication module 172 refers to a module for communication using electric signals or optical signals. Examples of wired communication techniques according to an embodiment may include a pair cable, a coaxial cable, an optical fiber cable, and an Ethernet cable.

The mobile communication module 173 transmits and receives wireless signals with at least one of a base station, an external terminal, and a server on a mobile communication network. Here, the wireless signals may include voice call signals, video call signals, or various types of data for transmission and reception of text/multimedia messages.

The memory 180 stores various data processed by the ultrasound diagnosis device 100. For example, the memory 180 may store medical data related to diagnosis of the object 10, such as ultrasound data and ultrasound image that are input or output, and may also store algorithms or programs to be executed in the ultrasound diagnosis device 100.

The memory 180 may be embodied as any of various storage media, e.g., a flash memory, a hard disk drive, an electrically erasable and programmable read only memory (EEPROM), etc. Furthermore, the ultrasound diagnosis device 100 may administer a web storage or a cloud server that performs a storage function of the memory 180 online.

The user input unit 190 generates data of an input that is made by the user to control operation of the ultrasound diagnosis device 100. The user input unit 190 may include hardware components, such as a keypad, a mouse, a touch pad, a trackball, and a jog switch. However, the user input unit 190 is not limited thereto, and may further include various other components, such as an electrocardiogram measuring module, a respiration measuring module, a voice recognition sensor, a gesture recognition sensor, a fingerprint recognition sensor, an iris recognition sensor, a depth sensor, and a distance sensor.

In particular, the user input unit 190 may include a touch screen in which a touch pad constitutes a layer structure together with the display unit 160 described above.

In this case, the ultrasound diagnosis device 100 according to an embodiment of the present invention may display an ultrasound image of a predetermined mode and a control panel for the ultrasound image on the touch screen. The ultrasound diagnosis device 100 may sense a touch gesture of the user for the ultrasound image through the touch screen.

The ultrasound diagnosis device 100 according to an embodiment of the present invention may physically include some buttons frequently used by users from among buttons included in a control panel of a general ultrasound device, and provide the other buttons through the touch screen in the form of a GUI.

The control unit 195 controls the overall operation of the ultrasound diagnosis device 100. In other words, the control unit 195 may control operations among the probe 20, the ultrasound transmission/reception unit 115, the image processing unit 150, the communication unit 170, the memory 180, and the user input unit 190 shown in FIG. 1.

All or some of the probe 20, the ultrasound transmission/reception unit 115, the image processing unit 150, the communication unit 170, the memory 180, the user input unit 190 and the control unit 195 may be operated by software modules. However, all or some of the components stated above may be operated not only by software modules but also by hardware modules. In addition, at least some of the ultrasound transmission/reception unit 115, the image processing unit 150, and the communication unit 170 may be included in the control unit 195. However, the present invention is not limited to the form of implementation.

FIG. 2 is a block diagram showing a configuration of an ultrasound diagnosis device 200 according to an embodiment of the present invention. Referring to FIG. 2, the ultrasound diagnosis device 200 may include an image generating unit 210, a parameter calculating unit 220, and a display unit 230.

The image generating unit 210, the parameter calculating unit 220, and the display unit 230 of FIG. 2 correspond to the image generating unit 155, the parameter calculating unit 130, and the display unit 160 of FIG. 1, respectively, and the same description will not be reiterated.

The image generating unit 210 may generate a first ultrasound image based on first ultrasound signals received from an object at a first point in time and a second ultrasound image based on second ultrasound signals received from the object at a second point in time. Here, the first point in time and the second point in time may be the same point in time in periods of the object.

The first and second ultrasound images may be 2D images or 3D images, and may correspond to each other.

The image generating unit 210 may perform surface rendering by using ultrasound data corresponding to the first ultrasound signals, thereby generating a 3D first ultrasound image. Also, to generate a 3D second ultrasound image in real time, the image generating unit 210 may perform volume rendering by using ultrasound data corresponding to the second ultrasound signals.

The parameter calculating unit 220 may calculate values of at least one parameter of the object based on the first and second ultrasound images. Parameters of an object have values that may indicate a state of the object. For example, when the object is the heart, parameters of the object may include a strain, a volume, a strain rate (SR), an ejection fraction, and a displacement.

The parameter calculating unit 220 may detect contour coordinates of the object included in the first and second ultrasound images and reference coordinates for calculating parameter values, and calculate parameter values based on the detected coordinates.

The display unit 230 may display a comparative image obtained by comparing the first and second ultrasound images, and display a comparative result obtained by comparing the parameter value calculated based on the first ultrasound image and the parameter value calculated based on the second ultrasound image.

The display unit 230 may display the first and second ultrasound images to overlap each other, and display a difference area between the first and second ultrasound images to be differentiated. For example, the difference area may be displayed in a different color from other areas.

Also, the display unit 230 may display the calculated parameter values in at least one of graphs, values, and colors, and display whether or not the calculated parameter values are within a normal range.

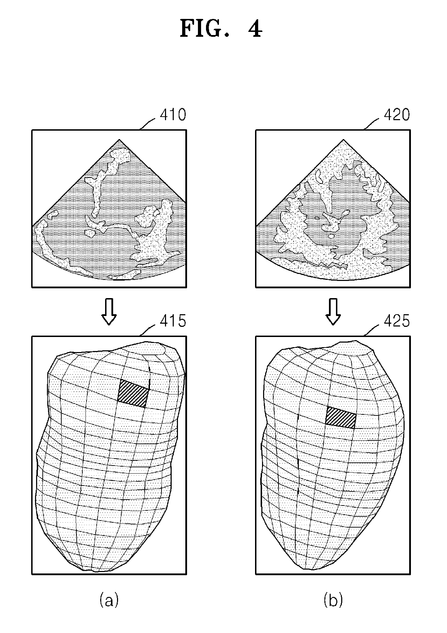

Operation of the display unit 230 will be described in detail later with reference to FIGS. 4 to FIG. 6.

Meanwhile, the block diagrams of the ultrasound diagnosis devices 100 and 200 shown in FIGS. 1 and 2 are for embodiments of the present invention. Respective components of the block diagrams may be integrated, added, or omitted according to specifications of an actually implemented ultrasound diagnosis device. In other words, in case of necessity, two or more components may be combined into one component, or one component may be subdivided into two or more components. Functions performed by each block are intended to describe embodiments of the present invention, and the detailed operation or unit of the block does not limit the scope of the present invention.

FIG. 3 is a flowchart of an operating method of an ultrasound diagnosis device according to an embodiment of the present invention.

Referring to FIG. 3, each of the ultrasound diagnosis devices 100 and 200 may generate a first ultrasound image of an object (operation 310).

For convenience of description, a case of the object being the heart will be described below as an example. However, the present invention is not limited to the case.

Each of the ultrasound diagnosis devices 100 and 200 may transmit ultrasound signals to the object and receive echo signals (first ultrasound signals) reflected by the object. Each of the ultrasound diagnosis devices 100 and 200 may process the first ultrasound signals, thereby obtaining a first ultrasound image of the object. Here, the first ultrasound image may be a B-mode image as shown in FIG. 4, but is not limited thereto.

The first ultrasound image may be a 2D ultrasound image or a 3D ultrasound image. Here, each of the ultrasound diagnosis devices 100 and 200 may perform surface rendering or volume rendering, thereby generating the 3D ultrasound image. The first ultrasound image may consist of different cross-sectional images that are generated based on ultrasound signals obtained in different directions from the object.

Meanwhile, the ultrasound diagnosis device 100 may store the generated first ultrasound image in the memory 180.

Each of the ultrasound diagnosis devices 100 and 200 may calculate a value of at least one parameter of the object based on the first ultrasound image (operation 320).

Parameters of an object have values that may indicate a state of the object. For example, parameters may have various measured values or values that are calculated based on the measured values.

When the object is the heart, parameters of the object may include a strain, a volume, an SR, an ejection fraction, and a displacement. Here, a value of a parameter may be calculated based on the first ultrasound image. For example, a value of a parameter of the object may be calculated based on coordinate values of the first ultrasound image. Also, the ultrasound diagnosis device 100 may store the value of the parameter calculated in this way in the memory 180.

Each of the ultrasound diagnosis devices 100 and 200 may generate a second ultrasound image (operation 330).

For example, each of the ultrasound diagnosis devices 100 and 200 may generate the second ultrasound image based on received second ultrasound signals, and the second ultrasound signals may be ultrasound signals received from the object to which a therapy is applied.

Here, when the object is the heart, the therapy may be cardiac resynchronization therapy (CRT). CRT is one of therapies for re-synchronizing heart muscles when the heart muscles are out of synchronization.

For example, when asynchronous ventricular contraction occurs in the heart, a capability for the heart to enter the systolic phase and the diastolic phase is reduced, and energy is inefficiently consumed. In other words, since premature ventricular contraction occurs at a portion of a ventricle and ill-timed ventricular contraction occurs at another portion of the ventricle, the cardiac output decreases, and the ventricular wall tension increases.

For this reason, CRT for synchronizing heart muscles may be performed on the heart which is out of synchronization. When CRT is performed, in order to determine whether or not the heart is regulated normally through CRT, it is necessary to compare in real time ultrasound images of the heart before and after CRT is performed, and also to compare quantitative values (parameter values) of the heart.

CRT for heart failure has been described above as an example, but the present invention is not limited thereto.

When various therapies are performed on objects other than the heart, in order to examine changes in the objects before and after performing the therapies or examine whether the therapies have effectively worked on the object, it is necessary to compare ultrasound images and parameter values.

For this reason, each of the ultrasound diagnosis devices 100 and 200 may transmit ultrasound signals to the object to which a therapy has been performed, and receive echo signals (second ultrasound signals) reflected by the object. Also, each of the ultrasound diagnosis devices 100 and 200 may receive the second ultrasound signals and generate a second ultrasound image in real time.

Each of the ultrasound diagnosis devices 100 and 200 may process the second ultrasound signals, thereby obtaining the second ultrasound image of the object. Like the first ultrasound image, the second ultrasound image may be a B-mode image as shown in (b) of FIG. 4, but is not limited thereto.

The second ultrasound image may be a 2D ultrasound image or a 3D ultrasound image. To generate the 3D ultrasound image in real time, each of the ultrasound diagnosis devices 100 and 200 may perform volume rendering by using ultrasound data corresponding to the second ultrasound signals. The second ultrasound image may consist of different cross-sectional images that are generated based on ultrasound signals obtained in different directions from the object.

Meanwhile, the second ultrasound image may be an image corresponding to the first ultrasound image so that the first and second ultrasound images may be readily compared with each other.

For example, when the first ultrasound image is a 2D image, the second ultrasound image may also be a 2D image, and when the first ultrasound image is a 3D image, the second ultrasound image may also be a 3D image.

In addition, the first and second ultrasound images may be images respectively based on first and second ultrasound signals obtained at the same point in time in periods of the object. For example, when the first ultrasound image is an ultrasound image obtained at a particular point in time of a cardiac systolic phase, the second ultrasound image may be an ultrasound image obtained at the particular point in time of another cardiac systolic phase.

Furthermore, when the first ultrasound image is a cross-sectional image obtained in a first direction, the second ultrasound image may also be a cross-sectional image obtained in the first direction.

Each of the ultrasound diagnosis devices 100 and 200 may calculate a value of the at least one parameter of the object based on the second ultrasound signals (operation 340).

As described above, parameters have values indicating characteristics of an object. Parameters may have various measured values or values that are calculated based on the measured values. For example, when the object is the heart, parameters of the object may include a strain, a volume, an SR, and a displacement.

Operation 340 corresponds to operation 320, and the same description will not be reiterated.

Each of the ultrasound diagnosis devices 100 and 200 may display a comparative image obtained by comparing the first and second ultrasound images and a comparative result obtained by comparing the calculated parameter values (operation 350).

Each of the ultrasound diagnosis devices 100 and 200 may separately display the first and second ultrasound images, compare the first and second ultrasound images, and display a difference area to be differentiated. Alternatively, each of the ultrasound diagnosis devices 100 and 200 may display the first and second ultrasound images to overlap each other so that the difference area may be differentiated.

For example, (a) of FIG. 4 shows a 2D image 410 and a 3D image 415 of a first ultrasound image, and (b) of FIG. 4 shows a 2D image 420 and a 3D image 425 of a second ultrasound image.

Since it is more difficult to recognize an area having a difference between 3D images than 2D images, the first 3D ultrasound image 415 and the second 3D ultrasound image 425 may be compared, and an area having a difference may be displayed to be differentiated. For example, the difference area may be displayed in a different color so that a user may readily recognize the difference area.

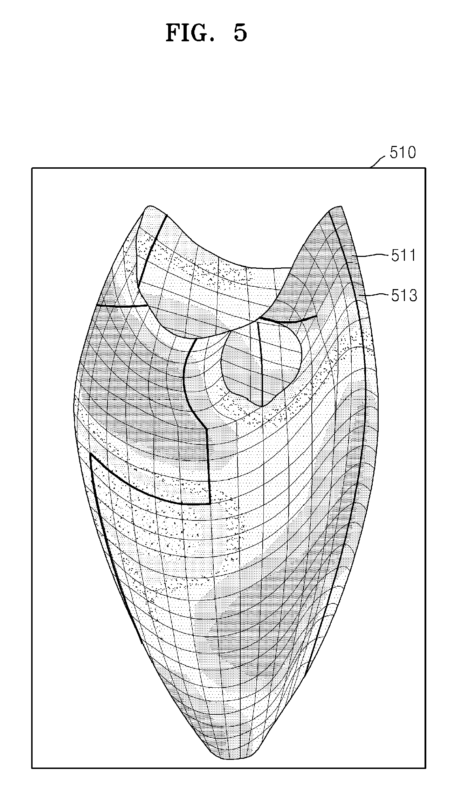

FIG. 5 shows an ultrasound image according to an embodiment of the present invention.

As shown in FIG. 5, each of the ultrasound diagnosis devices 100 and 200 may apply a color map based on improvement of an object to a second ultrasound image 510 and display the second ultrasound image 510 to which the color map has been applied.

Here, each of the ultrasound diagnosis devices 100 and 200 may compare normal parameter values of a fixed area in the object respectively with values of parameters (hereinafter, second parameters) calculated based on second ultrasound signals, thereby forming a color map.

For example, when a second parameter value is within a normal parameter value range, the fixed area may be displayed in a first color 511, and otherwise, the fixed area may be displayed in a second color 513.

Also, a ratio of a difference value between a second parameter value and a normal parameter value to the normal parameter value may be calculated, and the fixed area may be displayed in a different color. For example, the fixed area may be displayed in a different color according to a range of the ratio, such as a case where the fixed area is displayed in a first color when the calculated ratio is about 0% to less than about 5% and in a second color when the calculated ratio is about 5% to about 10%.

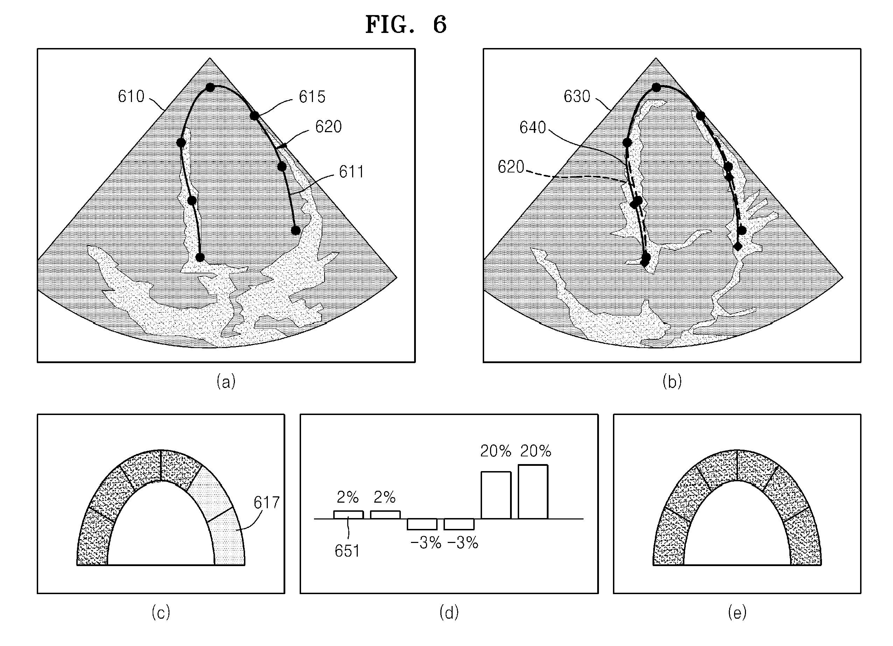

FIG. 6 shows ultrasound images and comparative results between the ultrasound images.

As shown in FIG. 6, each of the ultrasound diagnosis devices 100 and 200 may divide an object displayed in an ultrasound image into a plurality of sections, calculate a parameter value for each section, and display the parameter values in graphs, colors, values, and so on.

Referring to (a) of FIG. 6, each of the ultrasound diagnosis devices 100 and 200 may display a first ultrasound image 610 based on first ultrasound signals, and display a first reference curve 620 for dividing the object into a plurality of sections in the first ultrasound image. The first reference curve 620 may be divided into a plurality of sections. Here, reference points 615 may be displayed to distinguish the plurality of sections from each other, and the number of the reference points 615 may be set by a user input.

The plurality of sections may be divided automatically or based on a user input for selecting the reference points 615. For example, when a user makes an input for selecting first to n-th reference points, each of the ultrasound diagnosis devices 100 and 200 may set the distance between the first and second reference points as a first section, the distance between the second and third reference points as a second section, ..., and the distance between the (n-1)th and n-th reference points as an (n-1)th section.

An example in which first to sixth sections are set based on first to seventh reference points will be described below.

Each of the ultrasound diagnosis devices 100 and 200 may calculate a parameter value for a first section 611 based on the first ultrasound image 610, and display the calculated parameter value or a color corresponding to the calculated parameter value in a first area 617 corresponding to the first section 611 as shown in (c) of FIG. 6. For example, the first area 617 may be displayed in a first color when the calculated parameter value is within a normal range, and in a second color when the calculated parameter value is not within the normal range. Similarly, each of the ultrasound diagnosis devices 100 and 200 may calculate parameter values for second to sixth sections based on the first ultrasound image 610, and display the calculated parameter values or colors corresponding to the calculated parameter values in areas respectively corresponding to the sections.

Meanwhile, referring to (b) of FIG. 6, each of the ultrasound diagnosis devices 100 and 200 may display a second ultrasound image 630 based on second ultrasound signals and display a second reference curve 640 corresponding to the first reference curve 620. The second reference curve 640 represents a curve obtained through movement of the first reference curve 620 according to motion of the object.

For example, by tracking movement of a plurality of points included in the first reference curve 620, the second reference curve 640 may be detected. Also, as shown in (b) of FIG. 6, the first reference curve 620 may be displayed together in the second ultrasound image so that the degree of movement of the first reference curve 620 may be readily found.

Therefore, the second reference curve 640 may include reference points respectively corresponding to the first to seventh reference points of the first reference curve 620 shown in (a) of FIG. 6, and thus may include sections corresponding to the first to sixth sections of the first reference curve 620.

Each of the ultrasound diagnosis devices 100 and 200 may calculate parameter values for the first to sixth sections based on the second ultrasound image, and display the calculated parameter values or a color corresponding to the calculated parameter values in areas respectively corresponding to the first to sixth sections as shown in (e) of FIG. 6.

Meanwhile, each of the ultrasound diagnosis devices 100 and 200 may compare a value of the parameter (first parameter) calculated based on the first ultrasound image with a value of the parameter (second parameter) calculated based on the second ultrasound image, and display comparative results in graphs, colors, values, etc. in the display unit 160 or 230. For example, as shown in (d) of FIG. 6, a ratio of a difference value between the first parameter value and the second parameter value to the first parameter value may be displayed as a percentage according to the sections.

For example, as shown in (d) of FIG. 6, when the second parameter value of the first section increases by 2% compared to the first parameter value, a bar 651 in an area corresponding to the first section may be displayed in a size and a color corresponding to 2%, and the value may be displayed together. Also, bars in areas corresponding to the second to sixth sections may be displayed in the same way as described above.

As described above, comparative results of calculated parameter values are displayed in various ways, such as graphs, colors, values, etc., so that a user may readily compare parameter values of an object corresponding to first and second points in time.

In particular, it is possible to readily find a change in parameter values of an object between before and after a therapy, and a therapy may be efficiently performed on the object based on the change.

As described above, according to the one or more of the above embodiments of the present invention, a comparative image obtained by comparing ultrasound images of an object and a comparative result obtained by comparing parameter values are displayed, and thus a state change of the object may be accurately and readily diagnosed.

In addition, other embodiments of the present invention can also be implemented through computer readable code/instructions in/on a medium, e.g., a computer readable medium, to control at least one processing element to implement any above described embodiment. The medium can correspond to any medium/media permitting the storage and/or transmission of the computer readable code.

The computer readable code can be recorded/transferred on a medium in a variety of ways, with examples of the medium including recording media, such as magnetic storage media (e.g., ROM, floppy disks, hard disks, etc.) and optical recording media (e.g., CD-ROMs, or DVDs), and transmission media such as Internet transmission media. Thus, the medium may be such a defined and measurable structure including or carrying a signal or information, such as a device carrying a bitstream according to one or more embodiments of the present invention. The media may also be a distributed network, so that the computer readable code is stored/transferred and executed in a distributed fashion. Furthermore, the processing element could include a processor or a computer processor, and processing elements may be distributed and/or included in a single device.

It should be understood that the exemplary embodiments described therein should be considered in a descriptive sense only and not for purposes of limitation. Descriptions of features or aspects within each embodiment should typically be considered as available for other similar features or aspects in other embodiments.

While one or more embodiments of the present invention have been described with reference to the figures, it will be understood by those of ordinary skill in the art that various changes in form and details may be made therein without departing from the spirit and scope of the present invention as defined by the following claims.

## Claims

1. An operating method of an ultrasound diagnosis device, the operating method comprising:
generating a first ultrasound image based on first ultrasound signals received from an object at a first point in time;
calculating a value of at least one parameter of the object based on the first ultrasound image;
generating a second ultrasound image based on second ultrasound signals received from the object at a second point in time;
calculating a value of the at least one parameter of the object based on the second ultrasound image; and
displaying a comparative image obtained by comparing the first and second ultrasound images and a comparative result obtained by comparing the calculated values of the at least one parameter.

2. The operating method of claim 1, wherein the generating of the second ultrasound image comprises generating the second ultrasound image based on the second ultrasound signals received from the object to which a therapy has been applied.

3. The operating method of claim 2, wherein, when the object is a heart, the therapy is cardiac resynchronization therapy (CRT).

4. The operating method of claim 1, wherein the at least one parameter comprises at least one of a strain, a volume, a strain rate (SR), and a displacement.

5. The operating method of claim 1, wherein the first and second points in time are a same point in time in periods of the object.

6. The operating method of claim 1, further comprising storing the first ultrasound image and the value of the at least one parameter calculated based on the first ultrasound image,
wherein the generating of the second ultrasound image comprises receiving the second ultrasound signals and generating the second ultrasound image in real time, and
the displaying of the comparative image and the comparative result comprises displaying a comparative image obtained by comparing the stored first ultrasound image and the second ultrasound image generated in real time and a comparative result obtained by comparing the stored value of the at least one parameter and the value of the at least one parameter calculated based on the second ultrasound image.

7. The operating method of claim 1, wherein the generating of the first ultrasound image comprises generating a three-dimensional (3D) ultrasound image by performing surface rendering, and
the generating of the second ultrasound image comprises generating a 3D ultrasound image by performing volume rendering.

8. The operating method of claim 1, wherein the displaying of the comparative image and the comparative result comprises displaying the first and second ultrasound images to overlap each other, and displaying a difference area between the first and second ultrasound images to be differentiated.

9. The operating method of claim 8, wherein the displaying of the comparative image and the comparative result comprises displaying the difference area in a different color from other areas.

10. The operating method of claim 1, wherein the displaying of the comparative image and the comparative result comprises displaying the calculated values of the at least one parameter in at least one of graphs, values, and colors.

11. The operating method of claim 1, wherein the displaying of the comparative image and the comparative result comprises displaying whether or not the value of the at least one parameter calculated based on the second ultrasound image is within a normal range.

12. An ultrasound diagnosis device comprising:
an image generating unit which generate a first ultrasound image based on first ultrasound signals received from an object at a first point in time and a second ultrasound image based on second ultrasound signals received from the object at a second point in time;
a parameter calculating unit which calculates a value of at least one parameter based on the first ultrasound image and calculates a value of the at least one parameter based on the second ultrasound image; and
a display unit which displays a comparative image obtained by comparing the first and second ultrasound images, and displays a comparative result obtained by comparing the values of the at least one parameter.

13. The ultrasound diagnosis device of claim 12, wherein the image generating unit generates the second ultrasound image based on the second ultrasound signals received from the object to which a therapy has been applied.

14. The ultrasound diagnosis device of claim 13, wherein, when the object is a heart, the therapy is cardiac resynchronization therapy (CRT).

15. The ultrasound diagnosis device of claim 12, wherein the at least one parameter comprises at least one of a strain, a volume, a strain rate (SR), and a displacement.

16. The ultrasound diagnosis device of claim 12, wherein the first and second points in time are a same point in time in periods of the object.

17. The ultrasound diagnosis device of claim 12, further comprising a memory which stores the first ultrasound image and the value of the at least one parameter calculated based on the first ultrasound image,
wherein the image generating unit receives the second ultrasound signals and generates the second ultrasound image in real time, and
the display unit displays a comparative image obtained by comparing the stored first ultrasound image and the second ultrasound image generated in real time and a comparative result obtained by comparing the stored value of the at least one parameter and the value of the at least one parameter calculated based on the second ultrasound image.

18. The ultrasound diagnosis device of claim 12, wherein the image generating unit generates a three-dimensional (3D) first ultrasound image by performing surface rendering, and generates a 3D second ultrasound image by performing volume rendering.

19. The ultrasound diagnosis device of claim 12, wherein the display unit displays the first and second ultrasound images to overlap each other, and displays a difference area between the first and second ultrasound images to be differentiated.

20. The ultrasound diagnosis device of claim 19, wherein the display unit displays the difference area in a different color from other areas.

21. The ultrasound diagnosis device of claim 12, wherein the display unit displays the calculated values of the at least one parameter in at least one of graphs, values, and colors.

22. The ultrasound diagnosis device of claim 12, wherein the display unit displays whether or not the value of the at least one parameter calculated based on the second ultrasound image is within a normal range.

23. A computer-readable recording medium storing a program for performing the method of claim 1 in a computer.
